# EUROPÄISCHE PATENTSCHRIFT

(11) **EP 1 281 703 B1**
(45) Veröffentlichungstag und Bekanntmachung des Hinweises auf die Patenterteilung: **26.01.2005**
(21) Anmeldenummer: 02016604.7
(22) Anmeldetag: 25.07.2002
(51) Int. Cl.: C07D 263/42, C07D 213/67

(54) **Verfahren zur Herstellung von 5-Alkoxy-Oxazolen sowie von Pyridoxinen**
Process for the preparation of 5-alkoxy-oxazoles as well as of pyridoxines
Procédé pour la préparation de 5-alkoxy-oxazoles et de pyridoxines

(30) Priorität: 03.08.2001 DE 10137480; 05.03.2002 DE 10209446
(43) Veröffentlichungstag der Anmeldung: 05.02.2003
(73) Patentinhaber: BASF AKTIENGESELLSCHAFT, 67056 Ludwigshafen (DE)
(72) Erfinder: Rust, Harald, 67435 Neustadt (DE); Burkart, Kirsten, 67069 Ludwigshafen (DE); Faust, Tillmann, Dr., 67256 Weisenheim (DE); Henkelmann, Jochem, Dr., 68165 Mannheim (DE); Kindler, Alois, Dr., 67269 Grünstadt (DE); Knoll, Christian, Dr., 67141 Neuhofen (DE); Becker, Michael, Dr., 67063 Ludwigshafen (DE)

(56) Entgegenhaltungen:
- SU-A- 681 057
- DATABASE WPI Section Ch, Week 198017 Derwent Publications Ltd., London, GB; Class B03, AN 1980-30556C XP002217786 & SU 681 057 A (VITAMIN RES INST), 25. August 1979 (1979-08-25)
- DATABASE WPI Section Ch, Week 197933 Derwent Publications Ltd., London, GB; Class B03, AN 1979-60917B XP002217787 & JP 54 020493 B (DAIICHI PHARM CO LTD), 23. Juli 1979 (1979-07-23)

## Beschreibung

Die vorliegende Erfindung betrifft ein Verfahren zur Herstellung von 5-alkoxy-substituierten Oxazolen, insbesondere zur Herstellung von 4-Methyl-5-alkoxy-substituierten Oxazolen sowie ein Verfahren zur Herstellung von Pyridoxin-Derivaten.

5-alkoxy-substituierte Oxazole sind wertvolle Synthesebausteine in der organischen Chemie. Als wichtige Vorprodukte für die Synthese und industrielle Produktion von Vitamin B₆ haben 4-Methyl-5-alkoxy-substituierte Oxazole besondere Bedeutung (Turchi et al., Chem. Rev. 1975, 75, 416).

Ein wirtschaftliches und im großen Maßstab durchführbares Verfahren zur Herstellung von 5-alkoxy-substituierten Oxazolen, insbesondere von 4-Methyl-5-alkoxy-substituierten Oxazolen ist daher von hoher Bedeutung.

Es ist bekannt, α-Isocyanalkylsäureester diskontinuierlich durch thermische Isomerisierung in die entsprechenden 5-alkoxy-substituierten Oxazole umzuwandeln.

Itov et al., Khimiko-Farmatsevticheskii Zhurnal, 1978, 12, 102-106 und Mishchenlo et al., Khimiko-Farmatsevticheskii Zhurnal, 1988, 7, 856 bis 860 beschreiben eine diskontinuierliche, thermische Zyklisierung von α-Isocyanpropionsäureester zu den entsprechenden 4-Methyl-5-alkoxy-substituierten Oxazolen bei 135°C. Die durch Verwendung verschiedener Lösungsmittel erreichten Ausbeuten an 4-Methyl-5-alkoxy-substituierten Oxazolen liegen bei 4 bis 36 %. Das Verfahren weist den Nachteil einer geringen Selektivität und damit den Nachteil auf, dass große Mengen an Nebenprodukten entstehen. Häufigste Nebenprodukte dieser Reaktion sind das nicht umgesetzte Edukt (Ausbeute: 33 bis 55 %) sowie der umgelagerte α-Nitrilpropionsäureester (Ausbeute 1 bis 39 %).

Maeda et al., Bull. Chem. Soc. Japan, 1971, 44, 1407 bis 1410 offenbaren eine diskontinuierliche, thermische Zyklisierung von verschiedenen α-Isocyancarbonsäureestern zu den entsprechenden 5-alkoxy-substituierten Oxazolen bei Temperaturen von 150 bis 180°C. Je nach Substituenten werden Ausbeuten von 5,1 bis 28,2 % erreicht.

SU 682 057 beschreibt die Herstellung von 4-Methyl-5-Propoxyoxazol durch thermische Cyclisierung eines α- Isocyanpropionsäure-Propylesters. Zur Optimierung der Oxazol-Ausbeute werden sowohl das Lösungsmittel (DMF, HMPT, Acetonitril oder Triethylphosphat) als auch die Temperatur im Bereich von 135 bis 180°C variiert. Die höchste Ausbeute an 4-Methyl-5-Propoxyoxazol bezogen auf den eingesetzten α-Isocyanpropionsäure-Propylester wird in DMF bei 135°C erreicht und liegt bei lediglich 28 %.

In JP 54-20493 wird ein diskontinuierliches Verfahren zur Herstellung von 4-Methyl-5-alkoxy-substituierten Oxazolen durch thermische Zyklisierung von α-Isocyanpropionsäureester bei Temperaturen von 155 und 170°C in Gegenwart eines tertiären Amins beschrieben. Nach Beendigung der Reaktion wird die Lösung unter reduziertem Druck bei möglichst niedrigen Temperaturen fraktioniert destilliert. Dabei werden zwar verbesserte Selektivitäten an den gewünschten Oxazolen erreicht (34 bis 91,5 %) jedoch führt der geringe Umsatz (11,1 bis 49,4 %) noch zu keinen befriedigenden Ausbeuten.

Alle Verfahren des Standes der Technik weisen den Nachteil geringer Umsätze und geringer Selektivitäten und damit geringer Ausbeuten an 5-alkoxy-substituierten Oxazolen auf.

Der Erfindung lag daher die Aufgabe zugrunde, ein weiteres Verfahren zur Herstellung von 5-alkoxy-substituierten Oxazolen mit vorteilhaften Eigenschaften zur Verfügung zu stellen, das die Nachteile des Standes der Technik nicht mehr aufweist und die 5-alkoxy-substituierten Oxazole mit hohen Umsätzen in hohen Selektivitäten und Ausbeuten liefert.

Dementsprechend wurde ein Verfahren zur Herstellung von 5-alkoxy-substituierten Oxazolen der Formel I gefunden, 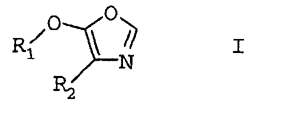 wobei
- R₁: einen, gegebenenfalls substituierten, C₁-C₆-Alkylrest und
- R₂: Wasserstoff oder einen, gegebenenfalls substituierten, C₁-C₆-Alkylrest
bedeuten,
indem man α-Isocyanalkylsäureester der Formel II 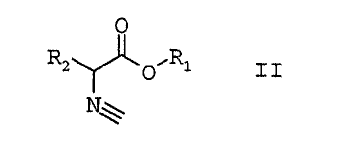 in Gegenwart von Gyclisierungshilfsstoffen ausgewählt aus der Gruppe der Basen, Alkohole oder Ester,
bei Temperaturen größer 80°C
in die 5-alkoxy-substituierten Oxazole der Formel I umwandelt
und gleichzeitig mit der Umwandlung die 5-alkoxy-substituierten Oxazole der Formel I aus dem Reaktionsgemisch abtrennt.

Unter einem gegebenenfalls substituierten, C₁-C6-Alkylrest werden für die Reste R₁ und R₂ unabhängig voneinander verzweigte oder unverzweigte, gegebenenfalls substituierten C₁-C₆-Alkylreste verstanden, wie beispielsweise gegebenenfalls substituiertes Methyl, Ethyl, Propyl, 1-Methylethyl, n-Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl.

Die Art der Substituenten ist nicht kritisch. Die C₁-C₆-Alkylreste können je nach freien Bindungsmöglichkeiten bis zu 6 Substituenten enthalten, vorzugsweise ausgewählt aus der Gruppe Aryl, Hydroxyaryl, -NO₂, -NH_{2,} -OH, -CN, -COOH, oder Halogen, insbesondere F oder Cl.

In einer bevorzugten Ausführungsform sind die C₁-C₆-Alkylreste der Reste R₁ und R₂ nicht substituiert.

Bevorzugte Reste für R₁ sind C₁-C₄-Alkyl-Reste, wie beispielsweise Methyl, Ethyl, iso-Propyl, n-Propyl, n-Butyl, sec-Butyl oder tert-Butyl, besonders bevorzugt n-Butyl.

Bevorzugte Reste für R₂ sind Wasserstoff und C₁-C₄-Alkyl-Reste, wie beispielsweise Methyl, Ethyl, iso-Propyl, n-Propyl, n-Butyl, sec-Butyl oder tert-Butyl, besonders bevorzugt Methyl.

Bevorzugt ist die Kombination der bevorzugten Reste für R₁ und R₂, besonders bevorzugt ist die Kombination R₁ = n-Butyl und R₂ = Methyl.

In einer besonders bevorzugten Ausführungsform des erfindungsgemäßen Verfahrens wird demnach α-Isocyanpropionsäure-n-butylester in 4-Methyl-5-n-Butoxy-Oxazol umgewandelt.

Die im erfindungsgemäßen Verfahren verwendeten α-Isocyanalkylsäureester der Formel II können in beliebiger Reinheit eingesetzt werden.

Die α-Isocyanalkylsäureester der Formel II können in an sich bekannter Weise aus den entsprechenden Formamidosäureestern der Formel V 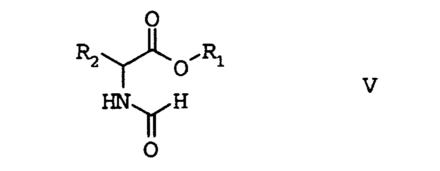 durch Umsetzung mit Phosphoroxychlorid oder Phosgen in Gegenwart von Basen hergestellt werden. Gängige Synthesemethoden sind in Itov et al., Khimiko-Farmatsevticheskii Zhurnal, 1978, 12, 102-106; Maeda et al., Bull. Chem. Soc. Japan, 1971, 44, 1407-1410; Ugi et al., Chem. Ber. 1961, 94, 2814; Chem. Ber. 1960, 93, 239-248, Angew. Chem. 1965, 77, 492-504, Chem. Ber. 1975, 1580-1590, DE 30 29 231 A1 und J. Heterocyclic Chemistry 1988, 17, 705 beschrieben.

Die Cyclisierungsreaktion erfolgt in Gegenwart von Cyclisierungshilfsstoffen, ausgewählt aus der Gruppe Basen, Alkohole oder Ester.

Unter Basen werden im erfindungsgemäßen Verfahren Verbindungen mit Brönsted-Basen-Eigenschaften verstanden. Bevorzugte Basen sind tertiäre Amine, wie beispielsweise Triethylamin, Triisopropylamin, Tri-n-Butylamin, Dimethylcyclohexylamin, Tris(2-ethylhexyl)amin, N-Methyl-pyrrolidon, N,N,N'N'-Tetramethyl-1,3-propandiamin, N,N-Diethylanilin oder N,N-Dibutylanilin. Besonders bevorzugt ist die Verwendung von Tri-n-butylamin als Base.

Bevorzugte Alkohole sind gegebenenfalls substituierte, C₁-C₆-Alkanole, wie beispielsweise Methanol, Ethanol, n-Propanol, isoPropanol, n-Butanol, sec-Butanol, tert-Butanol, n-Pentanol oder n-Hexanol. Besonders bevorzugt ist die Verwendung von n-Butanol als Alkohol.

Bevorzugte Ester sind gegebenenfalls substituierte C₁-C₆-Alkansäure-C₁-C₆-alkylester, wie beispielsweise Essigsäure-methylester, Essigsäure-ethylester, Essigsäure-propylester, Essigsäure-nbutylester, Essigsäure-tert-butylester, Essigsäure-hexylester, Propionsäure-methylester, Propionsäure-ethylester, Propionsäurepropylester, Propionsäure-n-butylester, Propionsäure-tert-butylester, Propionsäure-hexylester, Butansäure-methylester, Butansäure-ethylester, Butansäure-propylester, Butansäure-n-butylester, Butansäure-tert-butylester oder Butansäure-hexylester. Besonders bevorzugt ist die Verwendung von Propionsäure-n-butylester als Ester.

Die Cyclisierungshilfsstoffe können als einzelne Verbindungen oder auch in Form von Mischungen verwendet werden. Bevorzugt werden die Hilfsstoffe als einzelne Verbindungen verwendet.

Unter 80°C findet keine nennenswerte thermische Zyklisierung statt. Die Temperatur bei der erfindungsgemäßen Umwandlung beträgt daher mindestens 80°C.

In einer bevorzugten Ausführungsform erfolgt das erfindungsgemäße Verfahren bei Temperaturen von 100 bis 200°C, besonders bevorzugt bei Temperaturen von 120 bis 170°C, ganz besonders bevorzugt bei Temperaturen von 130 bis 170°C.

Das Molverhältnis von Cyclisierungshilfsstoff zu α-Isocyanalkylsäureester der Formel II ist nicht kritisch und beträgt vorzugsweise 10:1 bis 0,05:1.

Das erfindungsgemäße Verfahren lässt sich insbesondere diskontinuierlich, semidiskontinuierlich oder kontinuierlich durchführen.

In einer bevorzugten Ausführungsform des erfindungsgemäßen Verfahrens erfolgt die Durchführung des Verfahrens diskontinuierlich oder semidiskontinuierlich.

Unter diskontinuierlich wird eine batchweise Verfahrensführung verstanden. Dabei werden vorzugsweise die α-Isacyanalkylsäureester der Formel II und die Cyclisierungshilfsstoffe in einem Reaktor vorgelegt und die α-Isocyanalkylsäureester bei Temperaturen größer 80°C in die 5-alkoxy-substituierten Oxazole der Formel I umwandelt, wobei gleichzeitig mit der Umwandlung die 5-alkoxy-substituierten Oxazole der Formel I aus dem Reaktionsgemisch abgetrennt werden.

Es gibt viele Ausgestaltungen von Reaktoren, die für die bevorzugte diskontinuierliche Verfahrensweise in Frage kommen. Bevorzugte Reaktoren sollten die Eigenschaft aufweisen, eine Umwandlung bei gleichzeitiger Abtrennung eines Reaktionsprodukts zu ermöglichen.

Beispielsweise können als Reaktoren für die diskontinuierliche Verfahrensweise rückvermischte Reaktoren, wie beispielsweise Schlaufenreaktoren und übliche diskontinuierliche Reaktoren, wie beispielsweise Kessel in allen Ausgestaltungen, mit aufgesetzter Reaktionskolonne verwendet werden.

Unter semidiskontinuierlich wird eine semi-batchweise Verfahrensführung verstanden. Dabei werden vorzugsweise die α-Isocyanalkylsäureester der Formel II und die Cyclisierungshilfsstoffe semi-kontinuierlich einem Reaktor zugeführt und die α-Isocyanalkylsäureester bei Temperaturen größer 80°C in die 5-alkoxy-substituierten Oxazole der Formel I umwandelt, wobei gleichzeitig mit der Umwandlung die 5-alkoxy-substituierten Oxazole der Formel I aus dem Reaktionsgemisch abgetrennt werden.

Es gibt viele Ausgestaltungen von Reaktoren, die für die semi-diskontinuierliche Verfahrensweise in Frage kommen. Bevorzugte Reaktoren sollten die Eigenschaft aufweisen, eine Umwandlung bei gleichzeitiger Abtrennung eines Reaktionsprodukts zu ermöglichen.

Beispielsweise können als Reaktoren für die semidiskontinuierliche Verfahrensweise Schlaufenreaktoren, Membranreaktoren und übliche semi-diskontinuierliche Reaktoren, wie beispielsweise Kessel in allen Ausgestaltungen, mit aufgesetzter Reaktionskolonne verwendet werden.

In einer bevorzugten Ausführungsform führt man das Verfahren in einem diskontinuierlichen oder semidiskontinuierlichen Reaktor mit aufgesetzter Reaktionskolonne durch und trennt gleichzeitig mit der Umwandlung die 5-alkoxy-substituierten Oxazole der Formel I durch Rektifikation aus dem Reaktionsgemisch ab.

Wie dem Fachmann bekannt, werden nachstehend unter dem Begriff "Kolonne", wenn nicht anders erwähnt, ein Kolonnenaufbau mit Sumpf verstanden.

Unter einer "aufgesetzten Kolonne" wird dementsprechend nur der Kolonnenaufbau ohne Sumpf verstanden.

Unter Reaktionskolonnen bzw. aufgesetzten Reaktionskolonnen werden vorzugsweise Kolonnen verstanden, deren Einbauten einen hold-up aufweisen, also beispielsweise Kolonnen mit Böden, Schüttungen, Packungen oder Füllkörpern.

Besonders vorteilhafte Kolonnenböden ermöglichen eine hohe Verweilzeit der Flüssigkeit, wobei die Verweilzeit auf den Einbauten der Reaktionskolonne bevorzugt mindestens 30 min beträgt.

Bevorzugte Kolonnenböden sind beispielsweise Ventilböden, bevorzugt Glockenböden oder verwandte Bauarten wie zum Beispiel Tunnelböden, Lord-Stufen und andere Einbauten oder Thormannböden.

Bevorzugte strukturierte Packungen sind beispielsweise strukturierte Packungen vom Typ Mellapack® (Fa. Sulzer), BY® (Fa. Sulzer), B1® (Fa. Montz) oder A3® (Fa. Montz) oder Packungen mit vergleichbaren Ausgestaltungen.

Die Reaktionskolonnen bzw. aufgesetzten Reaktionskolonnen können von der Bauart und den Einbauten beliebig ausgestaltet sein. Besonders bevorzugt ist die Verwendung einer Trennwandkolonne als Reaktionskolonne.

Eine Reaktionskolonne bzw. aufgesetzten Reaktionskolonne, die auf verschiedenste Arten ausgestaltet sein kann, hat die Eigenschaft als Reaktor gleichzeitig eine Umwandlung von Reaktanten und die Abtrennung durch Rektifikation der 5-alkoxy-substituierten Oxazole der Formel I aus dem Reaktionsgemisch zu ermöglichen.

In dieser bevorzugten Ausführungsform der diskontinuierlichen oder semidiskontinuierlichen Verfahrensweise unter Verwendung eines diskontinuierlichen oder semidiskontinuierlichen Reaktors mit aufgesetzter Reaktionskolonne ist es weiterhin von Vorteil die Rektifikationsparameter so einzustellen, dass
- D: die Umwandlung der α-Isocyanalkylsäureester der Formel II in die 5-alkoxy-substituierten Oxazole der Formel I im Reaktor und/oder auf den Einbauten der aufgesetzten Reaktionskolonne erfolgt und
- E: die bei der Umwandlung entstehenden 5-alkoxy-substituierten Oxazole der Formel I über die aufgesetzte Reaktionskolonne abgetrennt werden.

Je nach Ausgestaltungsform des Reaktors, der aufgesetzten Reaktionskolonne und der verwendeten Reaktanten, wird dies durch unterschiedliche Einstellungen der Rektifikationsparameter erreicht. Geeignete Rektifikationsparameter sind beispielsweise Temperatur, Druck, Rücklaufverhältnis in der Kolonne, Ausgestaltung der Kolonne und deren Einbauten, Wärmeführung oder Energieeintrag, die der Fachmann durch Routineversuche so optimieren kann, dass die Merkmale D und E erreicht werden.

In der diskontinuierlichen und semidiskontinuierlichen Verfahrensweise wird der Druck am Kolonnenkopf der aufgesetzten Reaktionskolonne so eingestellt, dass die Temperatur im Reaktor und auf den Einbauten mindestens 80°C, vorzugsweise 100 bis 200°C, besonders bevorzugt 120 bis 170°C, ganz besonders bevorzugt 130 bis 170°C beträgt.

Typischerweise wird der Kopfdruck der Kolonne auf 5 bis 800 mbar eingestellt, so dass sich der daraus je nach verwendeter Kolonnenart und gegebenenfalls verwendeter Kolonneneinbauarten ergebende Sumpfdruck typischerweise 5 mbar bis atmosphärischer Druck beträgt.

Es ist möglich, dass die 5-alkoxy-substituierten Oxazole der Formel I mit den verwendeten Hilfsstoffen ein azeotropes Gemisch bilden, so dass die 5-alkoxy-substituierten Oxazole der Formel I über die aufgesetzte Kolonne als azeotropes Gemisch abgetrennt werden.

In diesem Fall ist es vorteilhaft, den Kopfdruck und damit auch automatisch den Sumpfdruck in der Kolonne, je nach hergestelltem 5-alkoxy-substituierten Oxazol der Formel I und verwendeter Hilfsstoffe so einzustellen, dass der Anteil an Cyclisierungshilfsstoff im Azeotrop im Kopfstrom möglichst gering ist.

Die Abtrennung der Cyclisierungshilfsstoffe aus dem Azeotrop erfolgt in diesem Fall in an sich bekannter Weise, beispielsweise durch eine sich anschließende zweite Rektifikation unter Verwendung eines anderen Drucks (Zweidruck-Destillation).

Beispielsweise bildet das nach dem erfindungsgemäßen Verfahren hergestellte 4-Methyl-5-n-Butoxy-Oxazol mit der Base Tri-n-Butylamin ein Azeotrop. Bei Einstellung eines Kopfdrucks von 100 mbar setzt sich das Azeotrop am Kopfstrom aus 91 Gew.-% 4-Methyl-5-n-Butoxy-Oxazol und 9 Gew.-% Tri-n-Butylamin zusammen.

Die Abtrennung von Tri-n-Butylamin aus dem Azeotrop kann in diesem Fall beispielsweise durch eine sich anschließende zweite Rektifikation bei einem Kopfdruck von 10 mbar erfolgen.

Die diskontinuierliche und semidiskontinuierliche Verfahrensweise kann in Anwesenheit oder Abwesenheit von Lösungsmitteln durchgeführt werden. In einer bevorzugten Ausführungsform erfolgt das Verfahren ohne Lösungsmittel.

Es ist jedoch möglich Lösungsmittel zuzusetzen oder Rohgemische im erfindungsgemäßen Verfahren einzusetzen, die neben den α-Isocyanalkylsäureester der Formel II und den Cyclisierungshilfsstoffen bereits Lösungsmittel enthalten.

In einer weiteren bevorzugten Ausführungsform erfolgt das erfindungsgemäße Verfahren in Gegenwart eines inerten Lösungsmittels. Unter einem inerten Lösungsmittel werden vorzugsweise unpolare und polare aprotische Lösungsmittel wie Toluol, Xylol oder Chlorbenzol, Dichlormethan, Dichlorethan, Dichlorbenzol, Ethylencarbonat, Propylencarbonat, insbesondere Chlorbenzol verstanden.

Dabei wird bei der diskontinuierlichen oder semidiskontinuierlichen Verfahrensweise zunächst das leichter siedende Lösungsmittel und anschließend die 5-alkoxy-substituierten Oxazol der Formel I durch Rektifikation abgetrennt.

In einer besonders bevorzugten Ausführungsform des erfindungsgemäßen Verfahrens erfolgt die Verfahrensweise kontinuierlich.

Bei der kontinuierlichen Verfahrensweise werden die α-Isocyanalkylsäureester der Formel II und die Cyclisierungshilfsstoffe entweder als Gemisch oder getrennt kontinuierlich einem Reaktor zugeführt, in dem Reaktor die α-Isocyanalkylsäureester der Formel II in die 5-alkoxy-substituierten Oxazole der Formel I umgewandelt und anschließend die Reaktionsprodukte kontinuierlich aus dem Reaktor entfernt, wobei gleichzeitig mit der Umwandlung die 5-alkoxy-substituierten Oxazole der Formel I kontinuierlich aus dem Reaktionsgemisch abgetrennt werden.

Es gibt viele Ausgestaltungen von Reaktoren, die für die besonders bevorzugte kontinuierliche Verfahrensweise in Frage kommen. Bevorzugte Reaktoren sollten die Eigenschaft aufweisen, eine kontinuierliche Umwandlung bei gleichzeitiger Abtrennung eines Reaktionsprodukts ermöglichen.

Beispielsweise können als Reaktoren für die kontinuierliche Verfahrensweise Blasen mit aufgesetzter Kolonne, Extraktionskolonnen, Glockenbodenkolonnen, Membranreaktoren, Lord-Reaktoren oder Reaktionskolonnen verwendet werden.

Wie vorstehend erwähnt, werden unter dem Begriff Kolonne, wenn nicht anders erwähnt, ein Kolonnenaufbau mit Sumpf verstanden.

Unter Reaktionskolonnen werden vorzugsweise Kolonnen verstanden, deren Einbauten einen hold-up aufweisen, also beispielsweise Kolonnen mit Böden, Schüttungen, Packungen oder Füllkörpern.

In einer besonders bevorzugten Ausführungsform des erfindungsgemäßen Verfahrens erfolgt die kontinuierliche Verfahrensweise in einer Reaktionskolonne als Reaktor, wobei gleichzeitig mit der Umwandlung die 5-alkoxy-substituierten Oxazole der Formel I kontinuierlich durch Rektifikation aus dem Reaktionsgemisch abtrennt.

Die Reaktionskolonnen können von der Bauart und den Einbauten beliebig ausgestaltet sein. Besonders bevorzugt ist die Verwendung einer Trennwandkolonne als Reaktionskolonne.

Eine Reaktionskolonne, die auf verschiedenste Arten ausgestaltet sein kann, hat die Eigenschaft als Reaktor gleichzeitig eine Umwandlung von Reaktanten und die Abtrennung durch Rektifikation der 5-alkoxy-substituierten Oxazole der Formel I aus dem Reaktionsgemisch zu ermöglichen.

In dieser besonders bevorzugten Ausführungsform unter Verwendung einer Reaktionskolonne für die kontinuierliche Verfahrensweise ist es weiterhin von Vorteil die Rektifikationsparameter so einzustellen, dass
- A: die Umwandlung der α-Isocyanalkylsäureester der Formel II in die 5-alkoxy-substituierten Oxazole der Formel I auf den Einbauten und gegebenenfalls im Sumpf der Reaktionskolonne erfolgt,
- B: die bei der Umwandlung entstehenden 5-alkoxy-substituierten Oxazole der Formel I kontinuierlich mit dem Kopfstrom oder Seitenstrom der Reaktionskolonne abgetrennt werden und
- C: der Cyclisierungshilfsstoff, sowie die bei der Umwandlung gegebenenfalls entstehenden Hochsieder kontinuierlich und unabhängig voneinander mit dem Sumpfstrom oder Seitenstrom der Reaktionskolonne abgetrennt werden.

Je nach Ausgestaltungsform der Reaktionskolonne und der verwendeten Reaktanten, wird dies durch unterschiedliche Einstellungen der Rektifikationsparameter erreicht. Geeignete Rektifikationsparameter sind beispielsweise Temperatur, Druck, Rücklaufverhältnis in der Kolonne, Ausgestaltung der Kolonne und deren Einbauten, Wärmeführung und Verweilzeit, insbesondere im Sumpf oder Energieeintrag, die der Fachmann durch Routineversuche so optimieren kann, dass die Merkmal A, B und C erreicht werden.

In Merkmal C kann der Cyclisierungshilfsstoff insbesondere auch getrennt von den Hochsiedern in einem zweiten Seitenstrom abgetrennt werden.

Unter Seitenstrom wird erfindungsgemäß der kontinuierliche Austrag einer Substanz über einen Seitenabzug der Kolonne verstanden.

Auch für die kontinuierliche Verfahrensweise des erfindungsgemäßen Verfahrens wird der Druck am Kolonnenkopf so eingestellt, dass die Temperatur im Sumpf und auf den Einbauten mindestens 80°C, vorzugsweise 100 bis 200°C, besonders bevorzugt 120 bis 170°C beträgt, ganz besonders bevorzugt 130 bis 170°C beträgt.

Typischerweise wird der Kopfdruck der Kolonne für die kontinuierliche Verfahrensweise auf 5 bis 800 mbar eingestellt, so dass sich der daraus je nach verwendeter Kolonnenart und gegebenenfalls verwendeter Kolonneneinbauarten ergebende Sumpfdruck typischerweise 5 mbar bis atmosphärischer Druck beträgt.

Die Verweilzeit in der Reaktionskolonne beträgt für die kontinuierliche Verfahrensweise typischerweise zwischen 10 Minuten und 7 Stunden, vorzugsweise zwischen 30 Minuten und 4 Stunden.

Es ist auch bei der kontinuierliche Verfahrensweise möglich, dass die 5-alkoxy-substituierten Oxazole der Formel I mit den verwendeten Cyclisierungshilfsstoffen ein azeotropes Gemisch bilden, so dass die 5-alkoxy-substituierten Oxazole der Formel I über den Kopfstrom als azeotropes Gemisch abgetrennt werden.

In diesem Fall ist es vorteilhaft, den Kopfdruck und damit auch automatisch den Sumpfdruck in der Kolonne, je nach hergestelltem 5-alkoxy-substituierten Oxazol der Formel I und verwendetem Hilfsstoff so einzustellen, dass der Anteil an Cyclisierungshilfsstoff im Azeotrop im Kopfstrom möglichst gering ist.

Die Abtrennung des Hilfsstoffs aus dem Kopfstrom-Azeotrop erfolgt in diesem Fall in an sich bekannter Weise, beispielsweise durch eine sich anschließende zweite Rektifikation unter Verwendung eines anderen Drucks (Zweidruck-Destillation).

Die kontinuierliche Verfahrensweise des erfindungsgemäßen Verfahrens kann in Anwesenheit oder Abwesenheit von Lösungsmitteln durchgeführt werden. In einer bevorzugten Ausführungsform erfolgt die kontinuierliche Verfahrensweise des erfindungsgemäßen Verfahren ohne Lösungsmittel.

In einer weiteren bevorzugten Ausführungsform erfolgt die kontinuierliche Verfahrensweise des erfindungsgemäßen Verfahrens in Gegenwart eines inerten Lösungsmittels. Unter einem inerten Lösungsmittel werden vorzugsweise unpolare und polare aprotische Lösungsmittel wie Toluol, Xylol oder Chlorbenzol, Dichlormethan, Dichlorethan, Dichlorbenzol, Ethylencarbonat, Propylencarbonat, insbesondere Chlorbenzol verstanden.

Im Falle der Verwendung eines Lösungsmittels kann das Lösungsmittel beispielsweise mit dem Cyclisierungshilfsstoff und den α-Isocyanalkylsäureester der Formel II im Gemisch oder jede einzelne Komponente separat kontinuierlich der Kolonne zugeführt werden.

Im Falle der Verwendung eines inerten Lösungsmittels in der kontinuierlichen Verfahrensweise des erfindungsgemäßen Verfahrens werden die Rektifikationsparameter vorzugsweise so einstellt, dass
- A: die Umwandlung der α-Isocyanalkylsäureester der Formel II in die 5-alkoxy-substituierten Oxazole der Formel I auf den Einbauten und gegebenenfalls im Sumpf der Reaktionskolonne erfolgt,
- B1: für den Fall, dass das Lösungsmittel einen höheren Siedepunkt als die bei der Umwandlung entstehenden 5-alkoxy-substituierten Oxazole der Formel I aufweist, die 5-alkoxy-substituierten Oxazole der Formel I kontinuierlich mit dem Kopfstrom und das Lösungsmittel kontinuierlich über den Seitenstrom oder Sumpfstrom der Reaktionskolonne abgetrennt werden,
- B2: für den Fall, dass das Lösungsmittel einen niedrigeren Siedepunkt als die die bei der Umwandlung entstehenden 5-alkoxy-substituierten Oxazole der Formel I aufweist, die 5-alkoxy-substituierten Oxazole der Formel I kontinuierlich mit einem Seitenstrom und das Lösungsmittel kontinuierlich mit dem Kopfstrom der Reaktionskolonne abgetrennt wird und
- C: der Cyclisierungshilfsstoff, sowie die bei der Umwandlung gegebenenfalls entstehenden Hochsieder kontinuierlich und unabhängig voneinander mit dem Sumpfstrom oder Seitenstrom der Reaktionskolonne abgetrennt werden.

Als Einbauten der Reaktionskolonne können jegliche Ausführungsformen verwendet werden, wie beispielsweise Kolonnenböden, Schüttungen, Füllkörper oder strukturierte Packungen.

Besonders vorteilhafte Kolonnenböden ermöglichen eine hohe Verweilzeit der Flüssigkeit, wobei die Verweilzeit auf den Einbauten der Reaktionskolonne bevorzugt mindestens 30 min beträgt.

Bevorzugte Kolonnenböden sind beispielsweise Ventilböden, bevorzugt Glockenböden oder verwandte Bauarten wie zum Beispiel Tunnelböden, Lord-Stufen und andere Einbauten oder Thormannböden.

Bevorzugte strukturierte Packungen sind beispielsweise strukturierte Packungen vom Typ Mellapack® (Fa. Sulzer), BY® (Fa. Sulzer), B1® (Fa. Montz) oder A3® (Fa. Montz) oder Packungen mit vergleichbaren Ausgestaltungen.

Das erfindungsgemäße Verfahren weist gegenüber dem Stand der Technik folgende Vorteile auf:

Mit dem erfindungsgemäßen Verfahren werden Selektivitäten von über 95 % bezogen auf den eingesetzten α-Isocyanalkylsäureester der Formel II erreicht.

Der Umsatz liegt bei nahezu 100 %, so dass die Ausbeute an 5-alkoxy-substituierten Oxazolen der Formel I über 95 % bezogen auf den eingesetzten α-Isocyanalkylsäureester der Formel II betragen.

Die besonders bevorzugte kontinuierliche Verfahrensweise weist den weiteren Vorteil einer deutlich größeren Raumzeitausbeute als bei den bisher bekannten Verfahren auf.

Das erfindungsgemäße Verfahren stellt einen neuen und vorteilhaften Teilsyntheseschritt im Verfahren zur Herstellung von Pyridoxin-Derivaten der Formel IX dar, 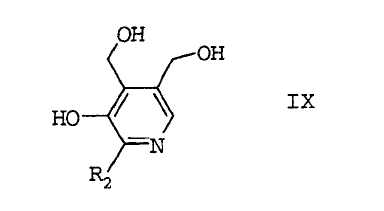 insbesondere zur Herstellung von Pyridoxin (Vitamin B₆; Formel IX, R₂ = Methyl).

Daher betrifft die Erfindung ferner ein Verfahren zur Herstellung von Pyridoxin-Derivaten der Formel IX
in dem man Aminosäuren der Formel III 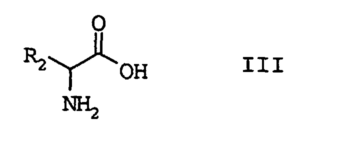 in Aminosäureester der Formel IV umwandelt, 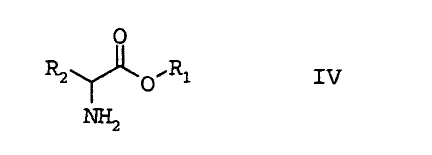 diese in Formamidosäureester der Formel V umwandelt, 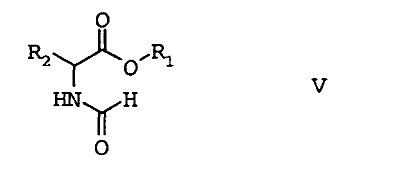 diese in α-Isocyanalkylsäureester der Formel II umwandelt, 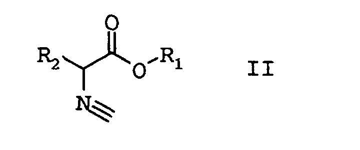 diese in Gegenwart von Cyclisierungshilfsstoffen ausgewählt aus der Gruppe Basen, Alkohole oder Ester
bei Temperaturen größer 80°C in die 5-alkoxy-substituierten Oxazole der Formel I umwandelt 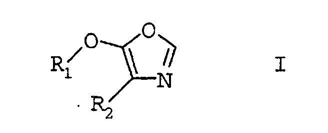 und gleichzeitig mit der Umwandlung die 5-alkoxy-substituierten Oxazole der Formel I aus dem Reaktionsgemisch abtrennt,
die 5-alkoxy-substituierten Oxazole der Formel I mit geschützten Diolen der Formel VI, 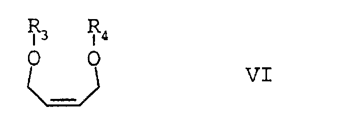 wobei
- R_{3,} R₄: unabhängig voneiunander oder R₃ und R₄ zusammen eine Schutzgruppe der Hydroxyfunktion
bedeuten,
zu den Diels-Alder-Addukten der Formel VII umsetzt, 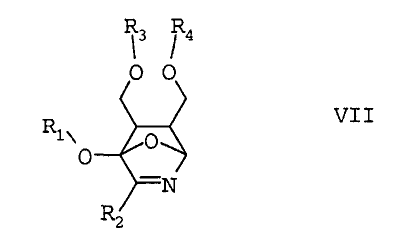 und diese durch saure Behandlung und Abspaltung der Schutzgruppe in die Pyridoxin-Derivate der Formel IX überführt.

Das Gesamtverfahren ist bis auf den erfindungsgemäßen neuen, vorteilhaften Teilschritt der Umwandlung von α-Isocyanalkylsäureester der Formel II in 5-alkoxy-substituierten Oxazole der Formel I aus Ullmann's Encyclopedia of Industrial Chemistry 1996, Vol. A 27, Seite 533 bis 537 bekannt.

Ausgangsstoffe für die Gesamtsynthese sind wohlfeile Aminosäuren der Formel III, vorzugsweise Alanin (R₂ = Methyl). Diese werden in an sich bekannter Weise, beispielsweise durch säurekatalysierte Veresterung mit den Alkoholen R₁-OH, vorzugsweise n-Butanol in Aminosäureester der Formel IV umgewandelt. Diese Veresterung kann aber auch durch andere Methoden, wie beispielsweise durch Aktivierung der Säurefunktion und basenkatalysierte Veresterung erreicht werden. Weiter Methoden sind in US 3,227,721 beschrieben.

Die Aminosäureester der Formel IV werden in an sich bekannter Weise, beispielsweise wie in US 3,227,721 beschrieben in die Formamidosäureester der Formel V umgewandelt.

Die Formamidosäureester der Formel V werden anschließend in an sich bekannter Weise, wie vorstehend beschrieben in die α-Isocyanalkylsäureester der Formel II umgewandelt.

Die α-Isocyanalkylsäureester der Formel II werden, wie vorstehend beschrieben mit dem erfindungsgemäßen Verfahren in die 5-alkoxy-substituierten Oxazole der Formel I umgewandelt.

Im bevorzugten Gesamtverfahren wird dieser Teilschritt in den bevorzugten Ausführungsformen, wie vorstehend beschrieben, durchgeführt.

Die 5-alkoxy-substituierten Oxazole der Formel I werden anschließend mit geschützten Diolen der Formel VI zu den Diels-Alder-Addukten der Formel VII umgesetzt.

Dieser Teilschritt kann dem erfindungsgemäßen Verfahren nachgeschaltet sein, kann aber bei der kontinuierlichen Verfahrensweise des erfindungsgemäßen verfahrens auch durch kontinuierliche Zuführung der geschützten Diolen der Formel VI zum Reaktor des erfindungsgemäßen Verfahrens gleichzeitig mit der Umwandlung der α-Isocyanalkylsäureester der Formel II in die 5-alkoxy-substituierten Oxazole der Formel I erfolgen. Die Zuführung erfolgt dabei entweder in Mischung mit den α-Isocyanalkylsäureester der Formel II, dem Cyclisierungshilfsstoff und gegebenenfalls dem Lösungsmittel oder als separate Komponente. Als Produkt werden in diesem Fall die 5-alkoxy-substituierten Oxazole direkt in Form ihres Diels-Alder-Addukts über den Sumpfaustrag der Kolonne entfernt.

Unter den Resten R₃, R₄ werden unabhängig voneiunander eine Schutzgruppe, vorzugsweise eine säurelabile Schutzgruppe der Hydroxyfunktion verstanden.

Prinzipiell kann jede säurelabile Schutzgruppe verwendet werden. Bevorzugte säurelabile Schutzgruppen sind die in der Literatur bekannten säurelabilen Schutzgruppen für Hydroxygruppen (T.W. Greene, Protective Groups in Organic Synthesis, John Wiley & Sons New York, 1981, Seite 14-71; P.J. Kocienski, Protecting Groups, Georg Thieme Verlag Stuttgart, 1994, Seite 21-94).

Ferner können, in einer bevorzugten Ausführungsform, die Reste R₃ und R₄ gemeinsam eine säurelabile Schutzgruppe der beiden Hydroxyfunktionen bilden. Vorzugsweise bilden dazu die beiden Hydroxyfunktionen mit Ketonen oder Aldehyden, wie beispielsweise Aceton oder Isobutyraldehyd ein cyclisches Acetal.

Durch anschließende saure Behandlung der Diels-Alder-Addukte der Formel VII erfolgt unter Abspaltung des Alkohols R₁-OH die Aromatisierung zum Pyridoxingerüst. Die Abspaltung der säurelabilen Schutzgruppe(n), die in der Regel durch saure, wässerige Behandlung erfolgt liefert die Pyridoxin-Derivaten der Formel IX, insbesondere Pyridoxin (Vitamin B6, R₂ = Methyl).

Der Alkohol R₁-OH und die Schutzgruppen R₃ und R₄ können wiedergewonnen und im Gesamtverfahren wieder eingesetzt werden.

Die Verwendung des erfindungsgemäßen neuen vorteilhaften Teilschritts im Gesamtverfahren führt zu einer Steigerung der Gesamtausbeute.

Die nachstehenden Beispiele erläutern die Erfindung

### Beispiel 1

### Kontinuierliche Herstellung von 4-Methyl-5-n-Butoxy-Oxazol in einer Trennwandkolonne

In eine kontinuierlich betriebene Trennwandkolonne (4,8 m x 64 mm) gefüllt mit 3 x 3 mm V₂A-Raschigringen und einer Trennwand von 2,4 m Höhe mit 60 theoretischen Trennstufen wurde ein Gemisch aus 20,5 Gew.-% α-Isocyanpropionsäure-n-butylester (R₁= n-Butyl, R₂ = Methyl) und 79,5 Gew.-% Tri-n-butylamin eingeleitet.

Bei 500 mbar Kopfdruck und einer Sumpftemperatur von 165°C geht 4-Methyl-5-n-Butoxy-Oxazol als Azeotrop mit Tri-n-butylamin (90:10 Gew.-%) mit einem Siedepunkt von 158°C über Kopf. Im Kolonnensumpf werden Hochsieder und Tributylamin abgezogen. Der Umsatz betrug 98,4 %, die Selektivität 99 %. Die Ausbeute an 4-Methyl-5-n-Butoxy-Oxazol betrug 95 % bezogen auf den eingesetzten α-Isocyanpropionsäure-n-butylester.

Das Azeotrop wurde anschließend in der gleichen Kolonne bei einem Kopfdruck von 10 mbar getrennt. Man erhält als Kopfprodukt ein Azeotrop mit der Zusammensetzung 4-Methyl-5-n-Butoxy-Oxazol:Trin-butylamin = 70:30 und im Seitenabzug reines 4-Methyl-5-n-Butoxy-Oxazol mit einem Siedepunkt von 98°C. Die Destillationsausbeute betrug 99 % (40 % reines 4-Methyl-5-n-Butoxy-Oxazol und 60 % 4-Methyl-5-n-Butoxy-Oxazol als Azeotrop, das in die erste Destillation zurückgeführt wurde). Das Rein-4-Methyl-5-n-Butoxy-Oxazol hatte einen Gehalt von 99,8 %.

### Beispiel 2

### Kontinuierliche Herstellung von 4-Methyl-5-n-Butoxy-Oxazol in einer Trennwandkolonne mit Lösungsmittel

In eine kontinuierlich betriebenen Trennwandkolonne (4,8 m x 64 mm) gefüllt mit 3 x 3 mm V₂A-Raschigringen und einer Trennwand von 2,4 m Höhe mit 60 theoretischen Trennstufen wurde ein Gemisch aus 13,1 Gew.-% α-Isocyanpropionsäure-n-butylester (R₁= n-Butyl, R₂ = Methyl), 32,2 Gew.-% Monochlorbenzol und 50,1 Gew.-% Tri-n-butylamin eingeleitet.

Bei 300 mbar Kopfdruck und einer Sumpftemperatur von 169°C geht mit einem Siedepunkt von 90°C Monochlorbenzol über Kopf, im Seitenabzug wird mit einer Übergangstemperatur von 151°C 4-Methyl-5-n-Butoxy-Oxazol als Azeotrop mit Tri-n-butylamin (88:12 Gew.-%) erhalten. Im Kolonnensumpf werden Hochsieder und Tributylamin abgezogen. Der Umsatz betrug 99,5 % die Selektivität 99 %. Die Ausbeute an 4-Methyl-5-n-Butoxy-Oxazol betrug 94 % bezogen auf den eingesetzten α-Isocyanpropionsäure-n-butylester.

Das Azeotrop wurde analog Beispiel 1 getrennt.

### Beispiel 3

### Kontinuierliche Herstellung von 4-Methyl-5-n-Butoxy-Oxazol in einer Reaktionskolonne mit Lösungsmittel

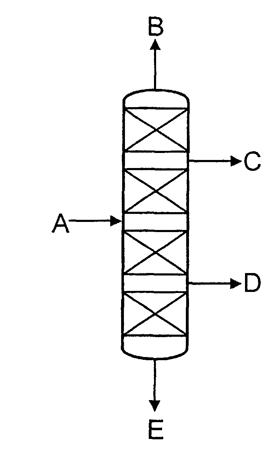

In einer Kolonne gemäß Beispiel 1 jedoch ohne Trennwand (siehe Abbildung 1) wurde über den Zulauf (A) ein Gemisch aus 20,6 Gew.-% Chlorbenzol, 5,2 Gew.-% α-Isocyanpropionsäuren-butylester (R₁= n-Butyl, R₂ = Methyl) und 72,60 Gew.-% Tris(2-ethylhexyl)amin kontinuierlich zugefahren.

Bei einem Kopfdruck von 300 mbar und einer Sumpftemperatur von 165°C geht das Lösungsmittel über Kopfabzug (B). Das 4-Methyl-5-n-Butoxy-Oxazol wird über den Seitenabzug (C) in einer Ausbeute von 99 % erhalten. Das Amin wird über den Sumpfaustrag (E) ausgeschleust.

### Beispiel 4

### Kontinuierliche Herstellung von 4-Methyl-5-n-Butoxy-Oxazol in einer Reaktionskolonne

Gemäß Beispiel 3 wird über den Zulauf (A) ein Gemisch aus 13,14 % α-Isocyanpropionsäure-n-butylester und 86,86 % Tris(2-ethylhexyl)amin kontinuierlich zugefahren.

Bei einem Kopfdruck von 400 mbar und einer Sumpftemperatur von 165°C wird das 4-Methyl-5-n-Butoxy-Oxazol über den Kopfabzug (B) ausgetragen und das Amin über den Sumpfaustrag (E) ausgeschleust. Die Ausbeute an 4-Methyl-5-n-Butoxy-Oxazol beträgt 98,8 %.

### Beispiel 5

### Kontinuierliche Herstellung von 4-Methyl-5-iso-Butoxy-Oxazol in einer Reaktionskolonne

Gemäß Beispiel 3 wird über den Zulauf (A) ein Gemisch aus 22,7 % α-Isocyanpropionsäure-iso-butylester und 77,3 % N,N-Dibutylanilin kontinuierlich zugefahren.

Bei einem Kopfdruck von 300 mbar und einer Sumpftemperatur von 160°C wird das 4-Methyl-5-iso-Butoxy-Oxazol bei einer Temperatur von 150°C über den Kopfabzug (B) ausgetragen. Das Amin wird über den Seitenabzug D bei 161°C erhalten. Die Ausbeute an 4-Methyl-5-iso-Butoxy-Oxazol beträgt 91 %.

### Beispiel 6

### Kontinuierliche Herstellung von 4-Methyl-5-n-Butoxy-Oxazol in einer Reaktionskolonne

Gemäß Beispiel 5 wird über den Zulauf (A) ein Gemisch aus 11,8 % α-Isocyanpropionsäure-n-butylester und 88,2 % N,N-Dibutylanilin kontinuierlich zugefahren. Man erhält 4-Methyl-5-n-Butoxy-Oxazol mit einer Ausbeute von 98,7 % über den Kopfabzug B und das Amin über den Seitenabzug D.

### Beispiel 7

### Kontinuierliche Herstellung von 4-Methyl-5-butyloxy-oxazol (MOX) in einer Reaktionskolonne unter Verwendung von n-Butanol als Cyclisierungshilfsstoff

In eine kontinuierlich betriebenen Trennwandkolonne (4,8 m x 64 mm) gefüllt mit 3 x 3 mm V₂A-Raschigringen und einer Trennwand von 2,4 m Höhe mit 60 theoretischen Trennstufen wurde ein Gemisch aus 30 Gew.-% α-Isocyan-propionsäure-*n*-butylester und 70 Gew.-% n-Butanol über den Zulauf (A) eingeleitet.

Bei einem Kopfdruck von 300 mbar und einer Sumpftemperatur von 165°C wird n-Butanol über Kopf abgetrennt. Der Kolonnensumpf wird mit Phthalsäure-di-n-butylester als Zwischensieder auf einem kontinuierlichen Stand gehalten.

Im Seitenabzug wird 4-Methyl-5-butyloxy-oxazol (MOX) als 97 % Reinstoff mit einer Ausbeute von 94 % erhalten.

### Beispiel 8

### Kontinuierliche Herstellung von 4-Methyl-5-butyloxy-oxazol (MOX) in einer Reaktionskolonne unter Verwendung von Propionsäure-n-butylester als Cyclisierungshilfsstoff

Die Apparatur und die experimentelle Durchführung entspricht Beispiel 7.

Analog zu Beispiel 7 wird ein Gemisch aus 35 Gew.-% α-Isocyanpropionsäure-n-butylester und 65 Gew.-% Propionsäure-n-butylester kontinuierlich eingeleitet.

Bei einem Kopfdruck von 300 mbar und einer Sumpftemperatur von 165°C wird Propionsäure-n-butylester über Kopf abgetrennt. Der Kolonnensumpf wird mit Phthalsäure-di-n-butylester als Zwischensieder auf einem kontinuierlichen Stand gehalten.

Im Seitenabzug wird 4-Methyl-5-butyloxy-oxazol (MOX) als 95 % Reinstoff mit einer Ausbeute von 92 % erhalten.

## Patentansprüche

1. Verfahren zur Herstellung von 5-alkoxy-substituierten Oxazolen der Formel I, 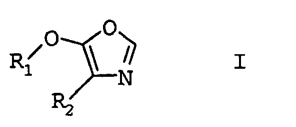 wobei
R₁ einen, gegebenenfalls substituierten, C₁-C₆-Alkylrest und
R₂ Wasserstoff oder einen, gegebenenfalls substituierten, C₁-C₆-Alkylrest
bedeuten,
indem man α-Isocyanalkylsäureester der Formel II 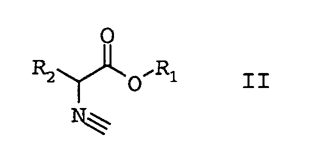 in Gegenwart von Cyclisierungshilfsstoffen, ausgewählt aus der Gruppe Basen, Alkohole oder Ester verwendet
bei Temperaturen größer 80°C
in die 5-alkoxy-substituierten Oxazole der Formel I umwandelt
und gleichzeitig mit der Umwandlung die 5-alkoxy-substituierten Oxazole der Formel I aus dem Reaktionsgemisch abtrennt.

2. Verfahren nach Anspruch 1, **dadurch gekennzeichnet, dass** man das Verfahren diskontinuierlich durchführt.

3. Verfahren nach Anspruch 2, **dadurch gekennzeichnet, dass** man das Verfahren in einem diskontinuierlichen oder semidiskontinuierlichen Reaktor mit aufgesetzter Reaktionskolonne durchführt und gleichzeitig mit der Umwandlung die 5-alkoxy-substituierten Oxazole der Formel I durch Rektifikation aus dem Reaktionsgemisch abtrennt.

4. Verfahren nach Anspruch 3, **dadurch gekennzeichnet, dass** man die Rektifikationsparameter so einstellt, dass
D die Umwandlung der α-Isocyanalkylsäureester der Formel II in die 5-alkoxy-substituierten Oxazole der Formel I im Reaktor und/oder auf den Einbauten der aufgesetzten Reaktionskolonne erfolgt und
E die bei der Umwandlung entstehenden 5-alkoxy-substituierten Oxazole der Formel I über die aufgesetzte Reaktionskolonne abgetrennt werden.

5. Verfahren nach einem der Ansprüche 2 bis 4, **dadurch gekennzeichnet, dass** man die Umwandlung in Gegenwart eines inerten Lösungsmittels durchführt.

6. Verfahren nach einem der Ansprüche 3 bis 5, **dadurch gekennzeichnet, dass** man als Reaktionskolonne eine Trennwandkolonne verwendet.

7. Verfahren nach einem der Ansprüche 3 bis 6, **dadurch gekennzeichnet, dass** man den Kopfdruck der Kolonne auf 5 bis 800 mbar einstellt und der sich daraus je nach verwendeter Kolonnenart und gegebenenfalls verwendeter Kolonneneinbautenart ergebende Sumpfdruck 10 mbar bis atmosphärischer Druck beträgt.

8. Verfahren nach Anspruch 1, **dadurch gekennzeichnet, dass** man das Verfahren kontinuierlich durchführt.

9. Verfahren nach Anspruch 8, **dadurch gekennzeichnet, dass** man das Verfahren in einer Reaktionskolonne durchführt und gleichzeitig mit der Umwandlung die 5-alkoxy-substituierten Oxazole der Formel I durch Rektifikation aus dem Reaktionsgemisch abtrennt.

10. Verfahren nach Anspruch 9, **dadurch gekennzeichnet, dass** man die Rektifikationsparameter so einstellt, dass
A die Umwandlung der α-Isocyanalkylsäureester der Formel II in die 5-alkoxy-substituierten Oxazole der Formel I auf den Einbauten und gegebenenfalls im Sumpf der Reaktionskolonne erfolgt,
B die bei der Umwandlung entstehenden 5-alkoxy-substituierten Oxazole der Formel I kontinuierlich mit dem Kopfstrom oder Seitenstrom der Reaktionskolonne abgetrennt werden und
C der Hilfsstoff, sowie die bei der Umwandlung gegebenenfalls entstehenden Hochsieder kontinuierlich und unabhängig voneinander mit dem Sumpfstrom oder Seitenstrom der Reaktionskolonne abgetrennt werden.

11. Verfahren nach einem der Ansprüche 8 bis 10, **dadurch gekennzeichnet, dass** man die Umwandlung in Gegenwart eines inerten Lösungsmittels durchführt und die Reaktionsparameter so einstellt, dass
A die Umwandlung der α-Isocyanalkylsäureester der Formel II in die 5-alkoxy-substituierten Oxazole der Formel I auf den Einbauten und gegebenenfalls im Sumpf der Reaktionskolonne erfolgt,
B1 für den Fall, dass das Lösungsmittel einen höheren Siedepunkt als die bei der Umwandlung entstehenden 5-alkoxy-substituierten Oxazole der Formel I aufweist, die 5-alkoxy-substituierten Oxazole der Formel I kontinuierlich mit dem Kopfstrom und das Lösungsmittel kontinuierlich über den Seitenstrom oder Sumpfstrom der Reaktionskolonne abgetrennt werden,
B2 für den Fall, dass das Lösungsmittel einen niedrigeren Siedepunkt als die bei der Umwandlung entstehenden 5-alkoxy-substituierten Oxazole der Formel I aufweist, die 5-alkoxy-substituierten Oxazole der Formel I kontinuierlich mit einem Seitenstrom und das Lösungsmittel kontinuierlich mit dem Kopfstrom der Reaktionskolonne abgetrennt wird und
C der Hilfsstoff, sowie die bei der Umwandlung gegebenenfalls entstehenden Hochsieder kontinuierlich und unabhängig voneinander mit dem Sumpfstrom oder Seitenstrom der Reaktionskolonne abgetrennt werden.

12. Verfahren nach einem der Ansprüche 9 bis 11, **dadurch gekennzeichnet, dass** man als Reaktionskolonne eine Trennwandkolonne verwendet.

13. Verfahren nach einem der Ansprüche 9 bis 12, **dadurch gekennzeichnet, dass** man, für den Fall dass der Hilfsstoff mit den 5-alkoxy-substituierten Oxazolen der Formel I ein Azeotrop bildet, den Kopfdruck in der Kolonne, so einstellt, dass der Anteil an Hilfsstoff im Azeotrop im Kopfstrom möglichst gering ist.

14. Verfahren nach einem der Ansprüche 9 bis 13, **dadurch gekennzeichnet, dass** man den Kopfdruck der Kolonne auf 5 bis 800 mbar einstellt und der sich daraus je nach verwendeter Kolonnenart und gegebenenfalls verwendeter Kolonneneinbautenart ergebende Sumpfdruck 10 mbar bis atmosphärischer Druck beträgt.

15. Verfahren zur Herstellung von Pyridoxin-Derivaten der Formel IX 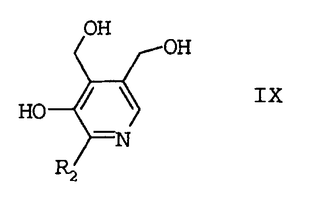 wobei
R₂ Wasserstoff oder einen, gegebenenfalls substituierten,C₁-C₆-Alkylrest bedeutet,
in dem man Aminosäuren der Formel III 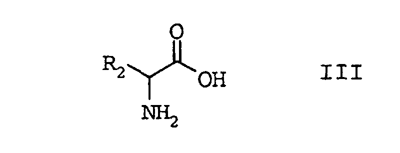 in Aminosäureester der Formel IV umwandelt, 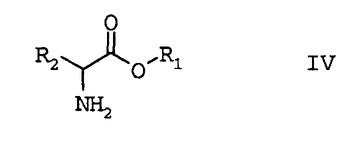 wobei
R₁ einen, gegebenenfalls substituierten, C₁-C₆-Alkylrest bedeutet,
diese in Formamidosäureester der Formel V umwandelt, 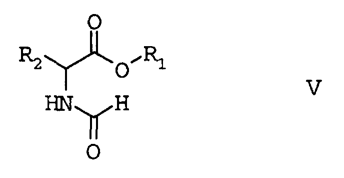 diese in α-Isocyanalkylsäureester der Formel II umwandelt, 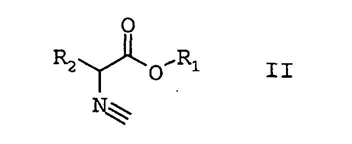 diese in Gegenwart von Cyclisierungshilfsstoffen, ausgewählt aus der Gruppe Basen, Alkohole oder Ester verwendet
bei Temperaturen größer 80°C
in die 5-alkoxy-substituierten Oxazole der Formel I umwandelt 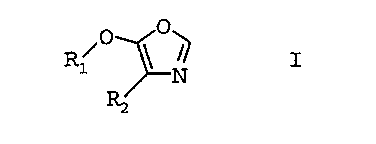 und gleichzeitig mit der Umwandlung die 5-alkoxy-substituierten Oxazole der Formel I aus dem Reaktionsgemisch abtrennt,
die 5-alkoxy-substituierten Oxazole der Formel I mit geschützten Diolen der Formel VI, 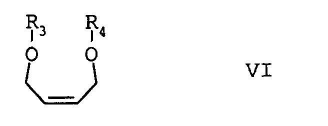 wobei
R_{3,} R₄ unabhängig voneiunander oder R₃ und R₄ zusammen eine Schutzgruppe der Hydroxyfunktion
bedeuten,
zu den Diels-Alder-Addukten der Formel VII umsetzt, 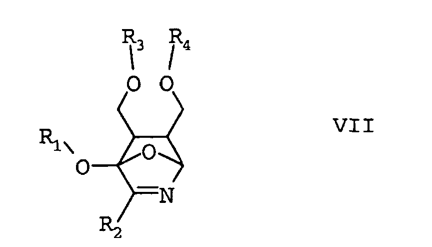 und diese durch saure Behandlung und Abspaltung der Schutzgruppe in die Pyridoxin-Derivaten der Formel IX überführt.

## Claims

1. A process for the preparation of 5-alkoxy-substituted oxazoles of the formula I, 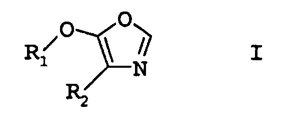 where
R₁ is an optionally substituted C₁-C₆-alkyl radical and
R₂ is hydrogen or an optionally substituted C₁-C₆-alkyl radical,
by converting α-isocyanoalkyl acid esters of the formula II 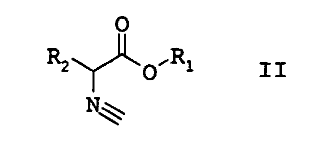 in the presence of cyclization assistants selected from the group consisting of bases, alcohols and esters
at temperatures of greater than 80°C
into the 5-alkoxy-substituted oxazoles of the formula I and, simultaneously to the conversion, separating the 5-alkoxy-substituted oxazoles of the formula I from the reaction mixture.

2. A process as claimed in claim 1, wherein the process is carried out batchwise.

3. A process as claimed in claim 2, wherein the process is carried out in a batchwise or semi-batchwise reactor having an attached reaction column and, simultaneously to the conversion, the 5-alkoxy-substituted oxazoles of the formula I are separated from the reaction mixture by rectification.

4. A process as claimed in claim 3, wherein the rectification parameters are adjusted such that
D the conversion of the α-isocyanoalkyl acid esters of the formula II into the 5-alkoxy-substituted oxazoles of the formula I takes place in the reactor and/or on the fittings of the attached reaction column and
E the 5-alkoxy-substituted oxazoles of the formula I formed during the conversion are separated by means of the attached reaction column.

5. A process as claimed in any of claims 2 to 4, wherein the conversion is carried out in the presence of an inert solvent.

6. A process as claimed in any of claims 3 to 5, wherein the reaction column used is a dividing wall column.

7. A process as claimed in any of claims 3 to 6, wherein the head pressure of the column is adjusted to 5 to 800 mbar and the bottom pressure resulting therefrom, depending on the type of column used and, if appropriate, the type of column fitting used, is 10 mbar to atmospheric pressure.

8. A process as claimed in claim 1, wherein the process is carried out continuously.

9. A process as claimed in claim 8, wherein the process is carried out in a reaction column and, simultaneously to the conversion, the 5-alkoxy-substituted oxazoles of the formula I are separated from the reaction mixture by rectification.

10. A process as claimed in claim 9, wherein the rectification parameters are adjusted such that
A the conversion of the α-isocyanoalkyl acid esters of the formula II into the 5-alkoxy-substituted oxazoles of the formula I takes place on the fittings and, if appropriate, in the bottom of the reaction column,
B the 5-alkoxy-substituted oxazoles of the formula I formed in the conversion are separated continuously with the head stream or side stream of the reaction column and
C the assistant, and also the high-boiling components which may be formed in the conversion are separated continuously and independently of one another with the bottom stream or side stream of the reaction column.

11. A process as claimed in any of claims 8 to 10, wherein the conversion is carried out in the presence of an inert solvent and the reaction parameters are adjusted such that
A the conversion of the α-isocyanoalkyl acid esters of the formula II into the 5-alkoxy-substituted oxazoles of the formula I takes place on the fittings and, if appropriate, in the bottom of the reaction column,
B1 in the case where the solvent has a higher boiling point than the 5-alkoxy-substituted oxazoles of the formula I formed in the conversion, the 5-alkoxy-substituted oxazoles of the formula I are separated continuously with the head stream and the solvent is separated continuously via the side stream or bottom stream of the reaction column,
B2 in the case where the solvent has a lower boiling point than the 5-alkoxy-substituted oxazoles of the formula I formed in the conversion, the 5-alkoxy-substituted oxazoles of the formula I are separated continuously with a side stream and the solvent is separated continuously with the head stream of the reaction column and
C the assistant, and also the high-boiling components which may be formed in the conversion are separated continuously and independently of one another with the bottom stream or side stream of the reaction column.

12. A process as claimed in any of claims 9 to 11, wherein the reaction column used is a dividing wall column.

13. A process as claimed in any of claims 9 to 12, wherein, in the case where the assistant forms an azeotrope with the 5-alkoxy-substituted oxazoles of the formula I, the head pressure in the column is adjusted such that the proportion of assistant in the azeotrope in the head stream is as low as possible.

14. A process as claimed in any of claims 9 to 13, wherein the head pressure of the column is adjusted to 5 to 800 mbar and the bottom pressure resulting therefrom, depending on the type of column used and, if appropriate, the type of column fitting used, is 10 mbar to atmospheric pressure.

15. A process for the preparation of pyridoxine derivatives of the formula IX 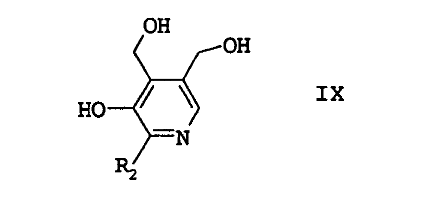 where
R₂ is hydrogen or an optionally substituted C₁-C₆-alkyl radical,
in which amino acids of the formula III 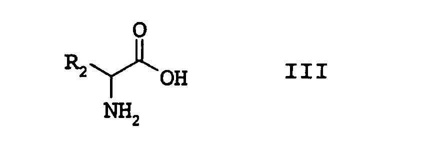 are converted into amino acid esters of the formula IV, 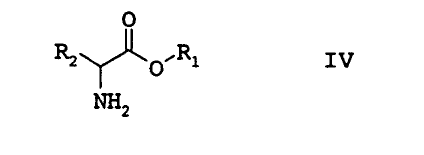 where
R₁ is an optionally substituted C₁-C₆-alkyl radical,
these are converted into formamido acid esters of the formula V 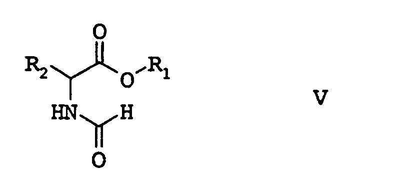 these are converted into α-isocyanoalkyl acid esters of the formula II, 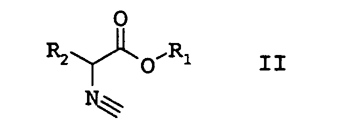 these are converted in the presence of cyclization assistants selected from the group consisting of bases, alcohols and esters
at temperatures of greater than 80°C
into the 5-alkoxy-substituted oxazoles of the formula I 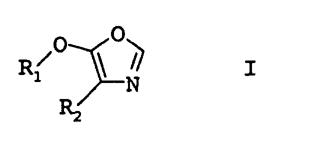 and, simultaneously to the conversion, the
5-alkoxy-substituted oxazoles of the formula I are separated from the reaction mixture,
the 5-alkoxy-substituted oxazoles of the formula I are reacted with protected diols of the formula VI, 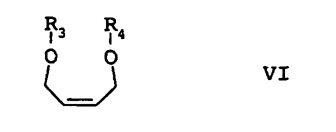 where
R₃, R₄ independently of one another or R₃ and R₄ together are a protective group of the hydroxyl function,
to give the Diels-Alder adducts of the formula VII, 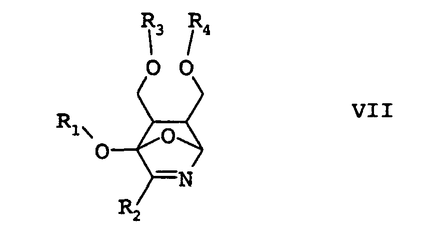 and these are converted into the pyridoxine derivatives of the formula IX by acid treatment and removal of the protective group.

## Revendications

1. Procédé de préparation d'oxazoles 5-alcoxy-substitués de la formule I : 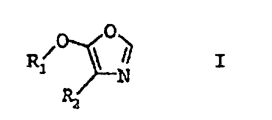 dans laquelle
R₁ représente un radical alkyle en C₁-C₆, éventuellement substitué, et
R₂ représente de l'hydrogène ou un radical alkyle en C₁-C₆, éventuellement substitué,
dans lequel on utilise des esters d'acide α-isocyanalkylique de la formule II : 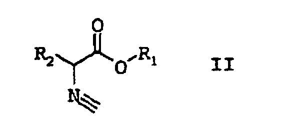 en présence de substances auxiliaires de cyclisation choisies parmi le groupe des bases, des alcools ou des esters,
on les convertit en les oxazoles 5-alcoxy-substitués de la formule I à des températures supérieures à 80°C, et simultanément à la conversion, on isole les oxazoles 5-alcoxy-substitués de la formule I à partir du mélange réactionnel.

2. Procédé suivant la revendication 1, **caractérisé en ce qu'**on effectue le procédé de manière discontinue.

3. Procédé suivant la revendication 2, **caractérisé en ce qu'**on effectue le procédé dans un réacteur discontinu ou semi-discontinu doté d'une colonne réactionnelle montée au-dessus et **en ce que**, simultanément à la conversion, on isole les oxazoles 5-alcoxy-substitués de la formule I par rectification à partir du mélange réactionnel.

4. Procédé suivant la revendication 3, **caractérisé en ce qu'**on ajuste les paramètres de rectification de façon que
D la conversion des esters d'acide α-isocyanalkylique de la formule II en les oxazoles 5-alcoxy-substitués de la formule I ait lieu dans le réacteur et/ou sur les éléments encastrés de la colonne réactionnelle montée au-dessus, et
E les oxazoles 5-alcoxy-substitués de la formule I, obtenus au cours de la conversion, soient isolés par l'intermédiaire de la colonne réactionnelle montée au-dessus.

5. Procédé suivant l'une des revendications 2 à 4, **caractérisé en ce qu'**on effectue la conversion en présence d'un solvant inerte.

6. Procédé suivant l'une des revendications 3 à 5, **caractérisé en ce que**, comme colonne réactionnelle, on utilise une colonne à paroi séparatrice.

7. Procédé suivant l'une des revendications 3 à 6, **caractérisé en ce qu'**on ajuste la pression de tête de la colonne à 5 jusqu'à 800 mbars et **en ce que** la pression de fond de colonne qui en résulte selon le type de colonne utilisé et le type d'éléments encastrés de colonne éventuellement utilisé est de 10 mbars à la pression atmosphérique.

8. Procédé suivant la revendication 1, **caractérisé en ce que** le procédé est effectué de manière continue.

9. Procédé suivant la revendication 8, **caractérisé en ce qu'**on effectue le procédé dans une colonne réactionnelle et **en ce que**, simultanément à la conversion, on isole les oxazoles 5-alcoxy-substitués de la formule I par rectification à partir du mélange réactionnel.

10. Procédé suivant la revendication 9, **caractérisé en ce qu'**on ajuste les paramètres de rectification de façon que
A la conversion des esters d'acide α-isocyanalkylique de la formule II en les oxazoles 5-alcoxy-substitués de la formule I ait lieu sur les éléments encastrés et éventuellement dans le fond de la colonne réactionnelle,
B les oxazoles 5-alcoxy-substitués de la formule I, obtenus au cours de la conversion, soient isolés de manière continue avec le courant de tête ou un courant latéral de la colonne réactionnelle, et
C la substance auxiliaire, ainsi que les substances à point d'ébullition élevé éventuellement formées au cours de la conversion soient isolées de manière continue et indépendamment l'une de l'autre avec le courant de fond ou un courant latéral de la colonne réactionnelle.

11. Procédé suivant l'une des revendications 8 à 10, **caractérisé en ce qu'**on effectue la conversion en présence d'un solvant inerte et **en ce qu'**on ajuste les paramètres réactionnels de façon que
A la conversion des esters d'acide α-isocyanalkylique de la formule II en les oxazoles 5-alcoxy-substitués de la formule I ait lieu sur les éléments encastrés et éventuellement dans le fond de la colonne réactionnelle,
B1 dans le cas où le solvant présente un point d'ébullition plus élevé que les oxazoles 5-alcoxy-substitués de la formule I obtenus au cours de la conversion, les oxazoles 5-alcoxy-substitués de la formule I soient isolés de manière continue avec le courant de tête et le solvant de manière continue par l'intermédiaire du courant latéral ou du courant de fond de la colonne réactionnelle,
B2 dans le cas où le solvant présente un point d'ébullition plus bas que les oxazoles 5-alcoxy-substitués de la formule I obtenus au cours de la conversion, les oxazoles 5-alcoxy-substitués de la formule I soient isolés de manière continue avec un courant latéral et le solvant de manière continue avec le courant de tête de la colonne réactionnelle, et
C la substance auxiliaire, ainsi que les substances à point d'ébullition élevé formées éventuellement au cours de la conversion soient isolées de manière continue et indépendamment l'une de l'autre avec le courant de fond ou un courant latéral de la colonne réactionnelle.

12. Procédé suivant l'une des revendications 9 à 11, **caractérisé en ce que**, comme colonne réactionnelle, on utilise une colonne à paroi séparatrice.

13. Procédé suivant l'une des revendications 9 à 12, **caractérisé en ce que**, dans le cas où la substance auxiliaire forme un azéotrope avec les oxazoles 5-alcoxy-substitués de la formule I, la pression de tête de la colonne est ajustée de façon que la fraction de substance auxiliaire dans l'azéotrope du courant de tête soit aussi faible que possible.

14. Procédé suivant l'une des revendications 9 à 13, **caractérisé en ce qu'**on ajuste la pression de tête de la colonne à 5 jusqu'à 800 mbars, et **en ce que** la pression de fond de colonne qui en résulte selon le type de colonne utilisé et le type d'éléments encastrés de colonne éventuellement utilisé est de 10 mbars à la pression atmosphérique.

15. Procédé de préparation de dérivés de pyridoxine de la formule IX : 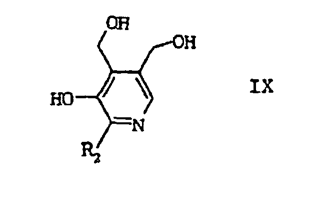 dans laquelle
R₂ représente de l'hydrogène ou un radical alkyle en C₁-C₆, éventuellement substitué,
dans lequel on convertit des acides aminés de la formule III : 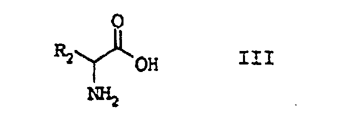 en esters d'acide aminé de la formule IV : 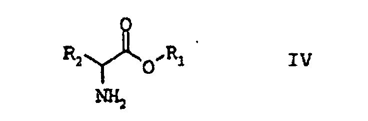 où
R₁ représente un radical alkyle en C₁-C₆, éventuellement substitué,
on convertit ceux-ci en esters de formamidoacide de la formule V : 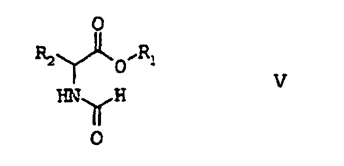 on convertit ceux-ci en esters d'acide α-isocyanalkylique de la formule II : 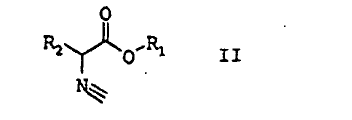 on utilise ceux-ci en présence de substances auxiliaires de cyclisation choisies parmi le groupe des bases, des alcools ou des esters,
on les convertit en les oxazoles 5-alcoxy-substitués de la formule I à des températures supérieures à 80°C 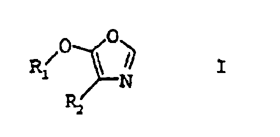 et, simultanément à la conversion, on isole les oxazoles 5-alcoxy-substitués de la formule I à partir du mélange réactionnel,
on fait réagir les oxazoles 5-alcoxy-substitués de la formule I avec des diols protégés de la formule VI : 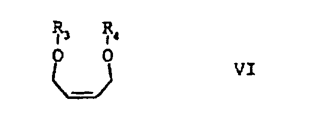 où
R₃, R₄ représentent indépendamment l'un de l'autre ou R₃ et R₄ conjointement un groupe de protection de la fonction hydroxy,
pour former les produits d'addition de Diels-Alder de la formule VII : 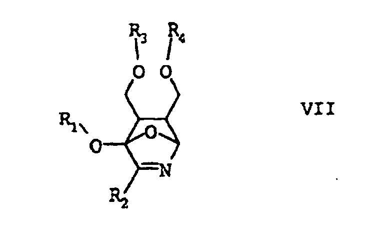 et on transforme ceux-ci, par traitement acide et élimination du groupe de protection, en les dérivés de pyridoxine de la formule IX.
